# EUROPÄISCHE PATENTSCHRIFT

(11) **EP 2 658 566 B1**
(45) Veröffentlichungstag und Bekanntmachung des Hinweises auf die Patenterteilung: **25.01.2017**
(21) Anmeldenummer: 11805055.8
(22) Anmeldetag: 27.12.2011
(51) Int. Cl.: A61K 38/20, C12N 15/113, A61P 35/00

(54) **SIRNA GEGEN CBL-B KOMBINIERT MIT ZYTOKINEN UND INTERFERONEN IN DER BEHANDLUNG VON KREBS**
SIRNA AGAINST CBL-B COMBINED WITH CYTOKINES AND INTERFERONS IN THE TREATMENT OF CANCER
SIRNA CONTRE LA CBL-B COMBINE AVEC DES CYTOKINES ET DES INTERFERONES DANS LE TRAITEMENT DU CANCER

(30) Priorität: 28.12.2010 EP 10197146
(43) Veröffentlichungstag der Anmeldung: 06.11.2013
(73) Patentinhaber: Apeiron Biologics AG, 1030 Wien (AT)
(72) Erfinder: LAMETSCHWANDTNER, Günther, A-1030 Wien (AT); LOIBNER, Hans, A-1230 Wien (AT); SCHUSTER, Manfred, A-2191 Schrick (AT); HASLINGER, Isabella, A-1030 Wien (AT); SEIDL, Sandra, A-1230 Wien (AT)
(74) Vertreter: Sonn & Partner Patentanwälte
(86) Internationale Anmeldenummer: PCT/EP2011/074099
(87) Internationale Veröffentlichungsnummer: WO 2012/089736

(56) Entgegenhaltungen:
- WO-A1-2010/119061
- WO-A2-2009/073905
- KOJO SATOSHI ET AL: "Mechanisms of NKT cell anergy induction involve Cbl-b-promoted monoubiquitination of CARMA1.", PROCEEDINGS OF THE NATIONAL ACADEMY OF SCIENCES OF THE UNITED STATES OF AMERICA 20 OCT 2009 LNKD- PUBMED:19815501, Bd. 106, Nr. 42, 20. Oktober 2009 (2009-10-20), Seiten 17847-17851, XP002635140, ISSN: 1091-6490 in der Anmeldung erwähnt
- LAMETSCHWANDTNER GUENTHER ET AL: "Development of an Effective Cancer Immune Therapy by Cbl-b Silencing", JOURNAL OF IMMUNOTHERAPY, LIPPINCOTT WILLIAMS & WILKINS, HAGERSTOWN, MD, US, Bd. 31, Nr. 9, 1. November 2008 (2008-11-01), Seite 943, XP008106012, ISSN: 1524-9557, DOI: DOI:10.1097/CJI.0B013E31818B1DCE in der Anmeldung erwähnt
- WIGGINTON JON M ET AL: "IL-12/IL-2 combination cytokine therapy for solid tumours: translation from bench to bedside.", EXPERT OPINION ON BIOLOGICAL THERAPY JUN 2002 LNKD- PUBMED:12079487, Bd. 2, Nr. 5, Juni 2002 (2002-06), Seiten 513-524, XP009152161, ISSN: 1471-2598
- JONATHAN M WEISS ET AL: "Immunotherapy of cancer by IL-12-based cytokine combinations", EXPERT OPINION ON BIOLOGICAL THERAPY, Bd. 7, Nr. 11, 1. November 2007 (2007-11-01), Seiten 1705-1721, XP055007188, ISSN: 1471-2598, DOI: 10.1517/14712598.7.11.1705
- ***: "Abstracts for the 25th Annual Scientific Meeting of theInternational Society for Biological Therapy of Cancer", JOURNAL OF IMMUNOTHERAPY, Bd. 33, Nr. 8, 1. Oktober 2010 (2010-10-01), Seiten 859-920, XP055007199, ISSN: 1524-9557, DOI: 10.1097/CJI.0b013e3181f1e08d
- INGUNN M. STROMNES ET AL: "Abrogating Cbl-b in effector CD8+ T cells improves the efficacy of adoptive therapy of leukemia in mice", JOURNAL OF CLINICAL INVESTIGATION, Bd. 120, Nr. 10, 1. Oktober 2010 (2010-10-01), Seiten 3722-3734, XP055007208, ISSN: 0021-9738, DOI: 10.1172/JCI41991
- VARIOUS: "International Society for Biological Therapy of Cancer 23rd Annual Meeting Abstracts", JOURNAL OF IMMUNOTHERAPY, Bd. 31, Nr. 9, 1. November 2008 (2008-11-01), Seiten 921-971, XP55027649,
- RICHARD ÅHLBERG ET AL: "Stimulation of T-cell cytokine production and NK-cell function by IL-2, IFN-[alpha] and histamine treatment during remission of non-Hodgkin's lymphoma", THE HEMATOLOGY JOURNAL, Bd. 4, Nr. 5, 1. Januar 2003 (2003-01-01), Seiten 336-341, XP55027663, ISSN: 1466-4860, DOI: 10.1038/sj.thj.6200320

## Beschreibung

Die vorliegende Erfindung betrifft therapeutische Verfahren zur Aktivierung des angeborenen Immunsystems, insbesondere von NK-Zellen.

NK-Zellen (natürliche Killerzellen) gehören zu den Lymphozyten (Untergruppe der weißen Blutzellen oder Leukozyten). Sie sind in der Lage abnormale Zellen, wie Tumorzellen und virusinfizierte Zellen, zu erkennen und abzutöten. NK-Zellen besitzen keine Antigen-spezifischen Rezeptoren und gehören zum angeborenen Immunsystem. NK-Zellen erkennen unter anderem den MHC-I-Komplex, der auf nahezu allen gesunden Körperzellen vorkommt. Wird eine Zelle durch Viren infiziert oder wandelt sie sich in eine Tumorzelle um, so geht unter Umständen der MHC-I-Komplex auf der Oberfläche verloren. NK-Zellen entwickeln sich wie die anderen Lymphozyten aus lymphatischen Vorläuferzellen im Knochenmark und zirkulieren später im Blutkreislauf.

Loeser et al. (JEM (2007) doi:10.1084/iem.20061699) zeigten, dass Cbl-b ein negativer Regulator ist, der maßgeblich für die "Immunreaktivität" von T-Zellen verantwortlich ist. Cbl-b unterdrückt die Aktivierung von T-Zellen und ist in der Lage Autoimmunreaktion zu verhindern. In Abwesenheit von Cbl-b können verabreichte aber kaum immunogene Substanzen zur Induktion einer starken Immunantwort führen. Ferner sind Cbl-b defiziente Mäuse (homozygotischer Gen knock-out) lebensfähig und deren Immunsystem ist in der Lage effizient autolog-induzierte Tumore zu erkennen und dagegen eine vor allem auf CD8+ T-Zellen-beruhende lytische Immunantwort aufzubauen. Allerdings führte die beschriebene, gänzliche Abschaltung des Enzyms ebenfalls zu einer erhöhten Autoimmunität nach Immunisierung mit Superantigenen.

Chiang et al. (Journal of Clinical Investigation 117 (4) (2007): 1033-1034) schreiben, dass Cbl-b⁻/⁻ CD8⁺ T-Zellen verwendet werden können, um die anti-Tumor-Reaktivität in E.G7-Mäusen zu erhöhen.

Kojo et al. (PNAS 2009; 106(42): 17847-17851) beschreibt einen Cbl-b beinflussten Mechanismus in einer künstlich ausgelösten Anergie von NKT-Zellen.

Die WO 2008/033403 A beschreibt die Erhöhung der Reaktivität von CD8+ T-Zellen durch Reduzierung der Cbl-b Aktivität. Inhibitorische RNA Sequenzen, insbesondere von siRNA, gegen Cbl-b wird offenbart.

WO 2009/073905 A2 beschreibt ein in vitro oder ex vivo Verfahren zur Erhöhung der Immunreaktion von Zellen des Immunsystems, welche mit einem Antigen kontaktiert wurden, umfassend die Reduzierung oder Inhibierung der Cbl-b Funktion dieser Zellen, wodurch die Immunreaktivität der Zellen gegen das Antigen erhöht wird.

WO 2010/119061 A1 betrifft Verfahren zur intrazellulären Bestimmung der Cbl-b Expression.

Lametschwandtner et al. (Journal of Immunotherapy 31 (9) (2008): 943) beschreiben Immuntherapien, welche auf der Unterdrückung von Cbl-b in T-Zellen beruhen.

Wigginton et al. (Expert Opinion on Biological Therapy 2002; 2(5): 513-524) beschreibt die Infiltration von Tumorgewebe durch CD8+ T-Zellen bei Gabe von IL-12 und IL-2.

Weiss et al. (Expert Opinion on Biological Therapy, 2007; 7(11): 1705-1721) beschreibt verschiedene Kombinationen von IL-12 mit weiteren Zytokinen als Wirkstoffe gegen Krebs.

Lametschwandtner et al. (J. of Immunotherapy 2010; 33(8): 899) beschreibt, dass die immunsteigernden Effekte von IL-7 auf humane T Zellen aufgrund einer Aktivierung des stat Signalweges, und nicht aufgrund einer Repression von Cbl-b erfolgen.

Stromnes et al. (J. of Clinical Investigation 2010; 120(10): 3722-3734) zeigt, dass die Verabreichung von ex vivo expandierten Cbl-b^{-/-} CD8 T-Zellen bei adoptiver Immuntherapie in Mäusen eine Antitumor-Reaktion in vivo auslöst.

Somit war bekannt, dass T-Zellen, also Zellen des adaptiven Immunsystems, mittels Cbl-b Inhibition aktiviert werden können um eine Immunantwort zu fördern. Eine solche Förderung der Immunantwort ist besonders für schwere chronische Krankheiten von therapeutischem Interesse, bei denen die nicht ausreichende Aktivität des Immunsystems kausal für den Verlauf der Erkrankung ist. Solche Erkrankungen sind beispielsweise chronische Infektionen oder Tumorerkrankungen. Allerdings bedürfen gerade effektive Immunantworten in diesen Erkrankungen eines effizienten Zusammenspiels des adaptiven und des angeborenen Immunsystems, wobei insbesondere zur Aktivierung von NK-Zellen noch keine ausreichenden Behandlungsansätze klinisch verfügbar sind.

Es ist daher ein vorrangiges Ziel neue Verfahren zu finden, welche durch die Aktivierung des angeborenen Immunsystems unter besonderer Bezugnahme auf NK-Zellen somit zu einer stark verbesserten Effektivität von Immunantworten führen können.

Erfindungsgemäß wurde dieses Ziel durch die Inhibition von Cbl-b in NK-Zellen gelöst.

Die Erfindung wird durch die unabhängigen Ansprüche definiert. Alle abweichenden Aspekte und Gegenstände werden hier nur für erläuternde Zwecke beschrieben, und Feststellungen, dass diese Teil der Erfindung o.ä. seien, müssen im Kontext der Anmeldung wie eingereicht gesehen werden und ändern den Schutzbereich der Ansprüche nicht.

In einem ersten Aspekt betrifft die Erfindung einen Cbl-b Inhibitor ausgewählt aus inhibierenden Nukleinsäuren, die die Expression von Cbl-b reduzieren oder inhibieren, in Kombination mit mindestens einem Zytokin oder Interferon ausgewählt aus
- IL-2 und einem oder mehreren weiteren Zytokinen oder Interferonen ausgewählt aus IL-12, IL-23, IFN-alpha und IFN-beta, oder
- IFN-alpha und einem oder mehreren weiteren Zytokinen ausgewählt aus IL-15 und IL-21, oder
- IL-12 und IL-7, oder
- IL-12 und IL-15, ist,
zur Verwendung in der therapeutischen Behandlung von Krebs in einem Patienten umfassend die in vivo Verabreichung des Cbl-b Inhibitors an einen Patienten und/oder die ex vivo Verabreichung der Zytokine und/oder Interferone an NK-Zellen des Patienten, und umfassend die Rückführung von ex vivo behandelten NK-Zellen in den Patienten.

"Verabreichung eines Cbl-b Inhibitors" oder "Cbl-b Inhibierung" sind Begriffe, die hierin, insbesondere bei der Beschreibung spezieller Ausführungsformen, austauschbar verwendet werden.

Die erfindungsgemäße Cbl-b Inhibitor Kombination kann für Immuntherapien in einem Patienten verwendet werden, insbesondere zur Aktivierung des Immunsystems bzw. des angeborenen Immunsystems, speziell vermittelt durch NK-Zellen. Insbesondere kann die Offenbarung zur Behandlung von Krebs, einer Virusinfektion, einer bakteriellen Infektion, insbesondere einer chronischen Infektion, im Besonderen einer chronische Infektion mit intrazellulär persistenten Bakterien, oder einer Parasiteninfektion, insbesondere einer Mykose, in dem Patienten eingesetzt werden. Die Therapie kann eine Immuntherapie einer chronischen Krankheit, inklusive chronischer Infektionen, sein. Die Infektion kann eines oder mehrere Organe betreffen, z.B. die Leber. Vorzugsweise ist die Infektion eine virale Infektion. Ein Beispiel ist chronische Hepatitis, z.B. ausgelöst durch eine Virusinfektion. Insbesonders bevorzugt ist die Behandlung von Hepatitis B oder Hepatitis C. Die erfindungsgemäße NK-Zell-Aktivierung mittels Cbl-b Inhibierung ist bei derartigen Krankheiten besonders effektiv. Der Patient ist vorzugsweise ein Säugetier, im speziellen ein Mensch.

Insbesondere im Fall von Krebs lässt sich die erfindungsgemäße NK-Zell-Aktivierung mit herkömmlichen Therapien kombinieren. Viele Tumortherapien, wie z.B. Strahlungstherapie, Chemotherapie oder operative Tumorentfernungen, sind seit Jahren etabliert und werden ständig verfeinert und verbessert. Neue Therapien umfassen Immuntherapien und Therapien, die auf spezifische Marker von Tumorzellen abzielen, insbesondere unter Verwendung von monoklonalen Antikörpern. Gerade die Wirkung letzterer hängt auch wesentlich von der Aktivität der NK-Zellen ab, die die tumorzellgebundenen Antikörper über generelle Antikörper-Determinanten erkennen und in weiterer Folge die Tumorzelle abtöten. Durch die Aktivierung des angeborenen Immunsystems über die Wirkung der NK-Zellen steht somit eine weitere Strategie zur Verfügung, die bisherige Ansätze ergänzen und vervollständigen kann um möglichst breit Immunreaktionen zu fördern, im Speziellen zur Bekämpfung von Krebszellen. Insbesondere Therapien, welche einen direkten zellschädigenden Einfluss auf die Tumorzellen haben, z.B. Chemotherapien oder Strahlentherapien, induzieren die Expression von Molekülen der MHC-Klasse und anderen immunaktivierenden Rezeptoren, bspw. von NKG2D-Liganden. Diese zellulären Veränderungen werden von Zellen des angeborenen Immunsystems, insbesondere NK-Zellen, erkannt und führen zu deren Aktivierung, welche durch die Synergie mit der erfindungsgemäßen NK-Zell-Aktivierung eine wesentlich höhere therapeutische Wirkung erzielen kann.

Demgemäß ist die erfindungsgemäß zu behandelnde Krebserkrankung vorzugsweise ausgewählt aus Krebserkrankungen des reproduktiven Traktes, insbesondere Eierstockkrebs, Hodenkrebs, Prostatakrebs oder Brustkrebs; Krebserkrankungen des Verdauungstraktes, insbesondere Magenkrebs, Darmkrebs, Rektalkarzinom, Pankreaskrebs, Speiseröhrenkrebs und Leberkrebs; Nierenkrebs, Hautkrebs, insbesondere Melanomen, Basalzellkarzinomen und Plattenepithelkarzinomen; Neuroblastomen und Glioblastomen, Lungenkrebs, Schilddrüsenkrebs, Sarkomen, Kopf-/Halskarzinom, Plattenepithelkarzinomen, Lymphomen und Leukämien (wobei hierin die Ausdrücke Krebs", "Tumor", "Karzinom", etc. immer synonym verwendet werden und sich auf bösartige Erkrankungen beziehen.

Das Cbl-b Gen sowie dessen Genprodukte sind im Stand der Technik ausführlich beschrieben (UniGene Id. Hs.3144 und Hs.381921). Cbl-b Sequenzen sind z.B. in der GenBank Datenbank unter den Acc. Nr. NM_008279 und NP_009112 veröffentlicht. Anti-Cbl-b Antikörper, siRNAs und Antisense-Inhibitoren sind kommerziell erhältlich. Bestimmte siRNAs geeignet zur Reduzierung oder Inhibierung der Cbl-b Expression und damit auch der Cbl-b Funktion sind beispielsweise in der US 2007/0054355 mit gemischten RNA/DNA Nukleotiden und einer Länge von ca. 20 Basen offenbart.

Cbl-b Inhibitoren sind im Stand der Technik hinlänglich bekannt. Beliebige Cbl-b Inhibitoren können gemäß der vorliegenden Erfindung eingesetzt werden. Der Cbl-b Inhibitor ist ausgewählt aus inhibierenden Nukleinsäuren, insbesondere antisense Oligonukleotiden, insbesondere antisense RNA, siRNA (small interfering RNA) oder shRNA (short hairpin RNA). Nukleinsäure Inhibitoren können als solche verwendet werden oder in Form von Vektoren, welche die Inhibierung kodieren und exprimieren. Geeignete Cbl-b Inhibitoren sind z.B. Antagonisten, Aptamere oder Intramere, vorzugsweise wird Cbl-b siRNA eingesetzt. siRNA Technologie zur Attenuierung der spezifischen Gen-Expression ist für Cbl-b bereits beschrieben. Cbl-b Inhibitoren in Sinne der vorliegenden Erfindung sind Substanzen, die die Expression und/oder die Funktion von Cbl-b reduzieren oder inhibieren und können, wie im Stand der Technik bekannt (Loeser et al. (JEM (2007) doi:10.1084/iem.20061699; Chiang et al. (Journal of Clinical Investigation 117 (4) (2007): 1033-1034); Lametschwandtner et al. (Journal of Immunotherapy 31 (9) (2008): 943); Paolini et al. (J Immunol. 2011 Feb 15;186(4):2138-47), oder wie in den vorliegenden Beispielen beschrieben, ermittelt werden.

Die US 2007/0087988 betrifft ein Verfahren zur Regulierung der HPK1, dessen Expression durch die Erhöhung der Cbl-b Expression erhöht werden kann und vice versa (z.B. durch Cbl-b siRNA Inhibition).

Vorzugsweise wird die Cbl-b Funktion durch Reduzierung oder Inhibierung der Expression von Cbl-b reduziert oder inhibiert. Die Begriffe Reduzierung oder Inhibierung betreffen die Absenkung der Funktion (bzw. der Expression) von Cbl-b im Vergleich zur unveränderten, natürlichen Funktion bis zur kompletten Inhibierung der Funktion. Vorzugsweise wird die Funktion (bzw. Expression) um mindestens 30%, 40%, 50%, 60%, 70%, 80%, 90% oder 95% reduziert.

Die Reduzierung oder Inhibierung der Funktion von Cbl-b erfolgt in vorzugsweisen Ausführungsformen transient. D.h. die Funktion wird nur vorläufig w.o. angegeben reduziert und kann sich in Folge dessen wieder erholen, z.B. durch Verbrauch oder Abbau von Inhibitoren, wie z.B. Cbl-b siRNA, oder durch Neubildung oder nicht Cbl-b beeinträchtigter Zellen in vivo. Die transiente Reduzierung von Cbl-b in Immunzellen kann dabei auch repetitiv erfolgen, z.B. bis ein therapeutischer Erfolg erzielt wurde.

Vorzugsweise wird die Expression von Cbl-b durch Verwendung von Cbl-b antisense RNA oder siRNA reduziert oder inhibiert. Hierzu werden kurze DNA und/oder RNA Sequenzen komplementär zu einem der Teile der Ziel (Cbl-b) mRNA Sequenz eingesetzt, so dass diese damit hybridisieren und inaktivieren. Die Länge dieser Sequenzen ist vorzugsweise mindestens 15, 18, 20, 22, 25, 28, 30, 35, 40, 50, 60, 70, 80, 90, 100, 120, 140, 160, 180 oder 200 Basen bis zur kompletten Ziel Sequenz, vorzugsweise bis zu 2500, 2000, 1500, 1000, 500 oder 300 Basen. Vorzugsweise werden die Sequenzen der SEQ ID Nr. 1, 2, 3, 4, 5, 6, 7 und/oder 8 verwendet.

Ebenfalls kann die Funktion von Cbl-b durch eine Vielzahl weiterer bekannter Komponenten reduziert oder inhibiert werden, wie beispielsweise durch Verwendung von Cbl-b Antagonisten, Inhibitoren, insbesondere Aptameren oder Intrameren. Jegliche Antagonisten oder Inhibitoren, die die Wirkung bzw. die Funktion von Cbl-b unterdrücken können erfindungsgemäß verwendet werden um die Immunreaktivität der NK-Zellen zu erhöhen. Zur Inhibition von Cbl-b können auch Substanzen verwendet werden, die entweder spezifisch die enzymatische E3-Ligase-Aktivität inhibieren oder die intrazelluläre Assoziation von Cbl-b mit seinen Interaktionspartner inhibieren oder die die Expression von Cbl-b inhibieren. Vorzugsweise werden Antagonisten oder Inhibitoren zur Herstellung eines pharmazeutischen Mittels zur erfindungsgemäßen Erhöhung der Immunreaktivität der NK-Zellen verwendet. Ermöglicht werden Behandlungen von Krankheiten mit supprimiertem oder ineffizientem Immunsystem, insbesondere Krebs oder chronische Infektionen.

Es wurde festgestellt, dass die Inhibierung von Cbl-b zusammen mit anderen NK-Zell-stimulierenden Substanzen (NK-Zell-Aktivatoren) einen synergistischen Effekt bewirkt, der über die zu erwartenden Effekte der additiven Effekte der Inhibition von Cbl-b und NK-Zell-Aktivierung hinausgeht. Die Verabreichung des Cbl-b Inhibitors bzw. die Cbl-b Inhibition erfolgt daher vorzugsweise zusammen mit einer weiteren NK-Zell stimulierenden Substanz (NK-Zell-Aktivator). Im folgenden werden die Begriffe "NK-Zell stimulierende Substanz", "NK-Zell aktivierende Substanz", und "NK-Zell-Aktivator" gleichbedeutend verwendet. Eine solche NK-Zell-stimulierende Substanz ist eine andere Substanz als der erfindungsgemäße Cbl-b Inhibitor. Eine NK-Zell stimulierende Substanz ist eine Substanz, die in einem oder mehreren geeigneten in-vitro Assays eine Aktivierung bzw. Stimulierung von NK-Zellen bewirkt. Vorzugsweise bewirkt die NK-Zell-stimulierende Substanz die Produktion von IFN-gamma, und/oder TNF-alpha, und/oder die CD107a Oberflächen-Expression, durch die NK-Zellen, unabhängig von einer Cbl-b Inhibierung. Eine solche Produktion von IFN-gamma, und/oder TNF-alpha, und/oder die CD107a Oberflächen-Expression, kann mit im Stand der Technik bekannten Methoden (Fauriat Blood. 2010 Mar 18;115(11):2167-76; Dons'koi et al., J Immunol Methods. 2011 Sep 30;372(1-2):187-95.), oder wie in den Beispielen der vorliegenden Erfindung beschrieben, gemessen werden. Ebenso kann die Wirkung der NK-Zell stimulierenden Substanzen durch die direkte Bestimmung der Zytotoxizität oder "killing activity" der NK-Zellen getestet werden (wie im Beispiel 4 beschrieben, weitere entsprechende Methoden sind im Stand der Technik bekannt (Beano et al., J Transl Med. 2008 May 16;6:25; Claus et al., J Immunol Methods. 2009 Feb 28;341(1-2):154-64; Fujisaki et al., Cancer Res. 2009 May 1;69(9):4010-7; Cho et al., Clin Cancer Res. 2010 Aug 1;16(15):3901-9), d.h. es wird die Zytotoxizität der NK-Zellen bzw. PBMCs auf bestimmte Ziel- bzw. Target-Zellen (im Beispiel 4 SKBR3-Tumorzellen) bestimmt, z. B. durch die Messung der Freisetzung des Enzyms LDH aus dem Tumorzellzytosol als Maß für die Zelllyse. Bei einer entsprechenden Messung in-vitro werden die NK-Zellen vorzugsweise aktiviert bzw. stimuliert, um einen Effekt der Cbl-b Inhibierung messen zu können, beispielsweise durch Kontakt mit Tumorzellen (z. B. K562), und/oder durch eine NK-Zell-stimulierende Substanz (beispielsweise ein oder mehrere Zytokine, wie z. B. IL-2 und/oder IL-12), und/oder einem Antikörper (z. B. Trastuzumab (Herceptin^{®})).

Offenbart wird die gemeinsame Verabreichung des Cbl-b Inhibitors mit einem NK-Zell-Aktivator, insbesondere ausgewählt aus einem Immunzell-stimulierenden Zytokin, bspw. aus einem Zytokin der common-gamma-chain Zytokine, insbesondere IL-2, IL-15, IL-21; Zytokinen, die sowohl Zellen des adaptiven als auch angeborenen Immunsystems stimulieren, insbesondere IL-12, IL-23, IL-27; Effektor-Zellzytokinen wie IL-1, IL-17, IL-18; einem Interferon, insbesondere Interferon-alpha; oder einem Interferon-Stimulans; einem Antikörper, insbesondere einem Antikörper, der Tumorzelloberflächenmoleküle erkennt, und/oder einem Antikörper dessen konstante Region an den entsprechenden Fc-Rezeptor an NK-Zellen binden kann; oder einem TLR- oder PAMP-Rezeptor Liganden, insbesondere Agonisten, vorzugsweise von TLR-1, TLR-2, TLR-3, TLR-7, TLR-8 oder TLR-9, sowie Kombinationen der oben genannten NK-Zell-Aktivatoren. Mit den Begriffen "gemeinsam" oder "zusammen mit" oder "in Kombination mit" oder "kombiniert mit" im Zusammenhang mit der Verabreichung der offenbarten Substanzen ist die Verabreichung mindestens eines Cbl-b Inhibitors und mindestens eines NK-Zell-Aktivators in einen Patienten definiert, die in Form von einer (enthält mindestens einen Cbl-b Inhibitor und mindestens einen NK-Zell-Aktivator) oder mehreren verschiedenen pharmazeutischen Zusammensetzungen (die eine enthält mindestens einen Cbl-b Inhibitor, eine andere mindestens einen NK-Zell-Aktivator, und optional weitere pharmazeutische Zusammensetzungen) erfolgen kann. Erfolgt die Verabreichung mittels mehrerer verschiedener pharmazeutischer Zusammensetzungen, kann die gemeinsame Verabreichung gleichzeitig oder zeitversetzt erfolgen. Insbesondere bevorzugt ist die Verabreichung des Cbl-b Inhibitors zusammen mit mindestens einem NK-Zell-Aktivator, insbesondere IL-2, IL-15, IL-12, IL-23, Interferon, einem Interferon-Stimulans, Imiquimod und andere TLR7/8-Agonisten, wie z.B. Resiquimod, ssPolyU-Nukleotide, Loxoribine, Gardiquimod, CL075, CL097, CL264, 3M002; poly(I:C) Oligonukleotide, CpG Oligonukleotide, CD205-Liganden oder CD206-Liganden, sowie Kombinationen hiervon. Bevorzugte Kombinationen von NK-Zell-Aktivatoren, die mit der Verabreichung des Cbl-b-Inhibitors kombiniert werden können, umfassen z.B. ein Zytokin der common-gamma-chain Zytokine mit einem anderen der obigen NK-Zell-Aktivatoren; oder ein Zytokin des adaptiven als auch angeborenen Immunsystems mit einem anderen der obigen NK-Zell-Aktivatoren. Erfindungsgemäße Kombinationen sind solche mit einem Zytokin der common-gamma-chain Zytokine und einem Zytokin des adaptiven als auch angeborenen Immunsystems, nämlich dass der Cbl-b Inhibitor in Kombination mit IL-2 und einem weiteren ausgewählt aus IL-12, IL-23, IFN-alpha und IFN-beta; in Kombination mit IFN-alpha und einem weiteren aus IL-15 und IL-21; in Kombination mit IL-12 und IL-7 oder in Kombination mit IL-12 und IL-15 verabreicht wird.

Ein Zytokin der common-gamma-chain Zytokine ist ausgewählt aus der Familie von Zytokinen, die die sogenannte gemeinsame Zytokin-Rezeptor gamma-Kette oder "common cytokine-receptor gamma-chain" (γ_{c} oder CD132) in ihren Rezeptor-Komplexen teilen, und besteht aus verschiedenen Mitgliedern mit einer ähnlichen Struktur mit vier alpha-Helices-Bündeln. Diese Familie umfasst z. B. Interleukin (IL)-2, IL-4, IL-7, IL-9, IL-15, IL-21 und "thymic stromal derived lymphopoietin" (TSLP). Ein Immunzellstimulierendes Zytokin, ein Zytokin des adaptiven als auch des angeborenen Immunsystems, ein Effektor-Zellzytokin, oder ein Interferon-Stimulans sind vorzugsweise ausgewählt aus der Gruppe umfassend IL-1, IL-2, IL-3, IL-4, IL-5, IL-6, IL-7, IL-8, IL-9, IL-11, IL-12, IL-13, IL-14, IL-15, IL-16, IL-17a, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IFN-alpha, IFN-beta, IFN-gamma, IFN-lambda, TNF-alpha, und TNF-beta.

Eine besonders bevorzugte Ausführungsform der Erfindung besteht in der Verabreichung eines Cbl-b Inhibitors zusammen mit IL-2, optional mit einem oder mehreren weiteren NK-Zell-Aktivatoren, insbesondere IL-12, IL-23, IFN-alpha und/oder IFN-beta. Eine weitere besonders bevorzugte Ausführungsform der Erfindung besteht in der Verabreichung eines Cbl-b Inhibitors zusammen mit IFN-alpha, optional mit einem oder mehreren weiteren NK-Zell-Aktivatoren, insbesondere IL-15 und/oder IL-21. Eine weitere besonders bevorzugte Ausführungsform der Erfindung besteht in der Verabreichung eines Cbl-b Inhibitors zusammen mit IL-12, optional mit einem oder mehreren weiteren NK-Zell-Aktivatoren, insbesondere IL-15 und /oder IL-7.

Vorzugsweise bewirken die eingesetzten NK-Zell-stimulierenden Substanzen in NK-Zellen eine IFN-gamma Produktion und/oder eine TNF-alpha Produktion, und/oder eine erhöhte CD107a Oberflächen-Expression, und/oder eine erhöhte Zytotoxizität auf Ziel- bzw. Target- Zellen. IFN-alpha, IL-12 oder IL-23 bewirken beispielsweise besonders starke IFN-gamma-Antworten in NK-Zellen. Überraschend wurde festgestellt, dass die Aktivierung von NK-Zellen durch Inhibierung von Cbl-b mit IFN-gamma-Produktion-verursachenden NK-Zell-stimulierenden Substanzen besonders starke synergistische Wirkungen hervorrufen, die weit über die zu erwartende Wirkung der Einzelsubstanzen hinausgeht.

Ebenso kann der Cbl-b Inhibitor zusammen mit einem Antikörper z. B. gegen Tumorzelldeterminanten verabreicht werden, optional kombiniert mit einem oder mehreren der oben angeführten Immunaktivatoren bzw. einer oder mehreren der oben genannten NK-Zell-stimulierenden Substanzen. Die Inhibition von Cbl-b in NK-Zellen als auch die Verabreichung des zusätzlichen NK-Zell-Aktivators bzw. des gegen Tumorzellen gerichteten Antikörpers können in vivo z.B. durch direkte Verabreichung an den Patienten erfolgen.

Vorzugsweise ist der Cbl-b Inhibitor an einen Liganden eines NK-Zell-Erkennungsmoleküls, z.B. eines NK-Zell-Oberflächenmoleküls, gekoppelt. Ein solcher Ligand kann bspw. ein natürlich vorkommendes Protein oder anderes Biomolekül sein oder ein funktionelles Derivat davon, welches NK-Zellen binden kann. Insbesondere kann ein solcher Ligand ein Antikörper gegen ein NK-Zellerkennungsmolekül sein. Erfindungsgemäß werden vorzugsweise spezifisch die NK-Zellen durch Cbl-b-Inhibierung aktiviert, z.B. durch Kopplung an einen Liganden eines solchen NK-Zell-Erkennungsmolekül, in vivo. Die "spezifische" NK-Zell Aktivierung soll als erhöhte Wirkung auf NK-Zellen verstanden werden im Vergleich zu unspezifischen, z.B. ungesteuerten bzw. ungekopppelten Cbl-b-Inhibitor Verabreichungen, welche auch in anderen Zellen Wirkung entfalten können. Insbesondere soll die "spezifische" NK-Zell Aktivierung als Wirkung insbesondere auf NK-Zellen verstanden werden im Vergleich zur Verabreichungen eines Cbl-b Inhibitors alleine (ohne die Verabreichung zusammen mit einem NK-Zell-Aktivator), oder im Vergleich zur Verabreichung eines Cbl-b-Inhibitors, der nicht an einen Liganden eines NK-Zell-Erkennungsmoleküls gekoppelt ist. Eine unspezifische Verabreichung erfolgt z.B. durch einfache Verabreichung des Inhibitors ohne zusätzliche Verabreichung von NK-Zell-Stimulantien oder NK-zell-spezifische Modifikationen des Inhibitors durch Kopplung an ein NK-Zell-Erkennungsmolekül. Durch die spezifische NK-Zell Aktivierung lässt sich gezielt die erfindungsgemäße NK-Zell-vermittelte Immunantwort (angeborenes Immunsystem) steuern, mit geringeren oder ungewünschten Nebenwirkungen, z.B. durch das adaptive Immunsystem.

Unter dem Begriff "Antikörper" werden alle natürlich vorkommenden Antikörper verstanden, wie IgG-, IgD-, IgA-, IgE-, IgM-Antikörper, sowie deren funktionelle Derivate, welche beispielsweise Fab-, Fab'-, F(ab)₂-, F(ab')₂-Fragmente, einzelkettige Antikörper (scAb oder scFv), oder ein Fragment einer variablen Antikörperdomäne, umfassen und welche ein Antigen, hier insbesondere ein NK-Zell-Erkennungsmolekül, oder den entsprechenden Fc-Rezeptor an NK-Zellen spezifisch binden bzw. dagegen gerichtet sind. Erfindungsgemäße Antikörper besitzen vorzugsweise eine konstante Region, insbesondere eine Fc-Domäne, die an den entsprechenden Fc-Rezeptor an NK-Zellen binden kann. Der Antikörper kann monoklonal oder polyklonal sein. Antikörper, welche wie zuvor beschrieben in einer Therapie in Kombination mit dem erfindungsgemäßen Cbl-b-Inhibitor verabreicht werden sollen, sind gegen ein Epitop eines Krankheitserregers gerichtet, oder ein Tumorepitop, und weisen vorzugsweise eine Fc-Domäne auf.

Zu den bevorzugten NK-Zellerkennungsmolekülen gehören insbesondere solche die entweder spezifisch nur auf NK-Zellen vorkommen oder die besonders häufig auf NK-Zellen exprimiert sind und gegebenenfalls zusätzlich noch auf weiteren Immunzellen deren Aktivität wunschgemäß durch Cbl-b Inhibierung erhöht werden kann bspw. CD2, CD8a, CD16, CD25, CD27, CD56, CD71, CD158, CD159, CD160, CD161, CD205, CD206, CD205, CD314, CD335, CD336, CD337. Dadurch kann der Cbl-b Inhibitor entweder spezifisch an NK-Zellen oder an NK-Zellen und relevante weitere Subtypen von Immunzellen in einem Patienten gezielt herangebracht und durch die Zellen aufgenommen werden. Damit kann bewirkt werden, dass nur spezifisch in den gewünschten Ziel-Zellen der Cbl-b Inhibitor therapeutisch wirksam wird.

In einem weiteren Aspekt betrifft die vorliegende Erfindung eine pharmazeutische Zusammensetzung umfassend einen Cbl-b Inhibitor ausgewählt aus inhibierenden Nukleinsäuren, die die Expression von Cbl-b reduzieren oder inhibieren, und
- IL-2 und ein oder mehrere weitere Zytokine oder Interferone ausgewählt aus IL-12, IL-23, IFN-alpha und IFN-beta, oder
- IFN-alpha und ein oder mehrere weitere Zytokine ausgewählt aus IL-15 und IL-21, oder
- IL-12 und IL-7, oder
- IL-12 und IL-15,
zur Verwendung in der therapeutischen Behandlung von Krebs in einem Patienten.

In einer weiteren bevorzugten Ausführungsform umfasst die Zusammensetzung IL-2 und IL-12, oder IL-2 und IFN-beta, oder IL-2 und IL-23, oder IL-12 und IL-7, oder IL-12 und IL-15, oder IFN-alpha und IL-2, oder IFN-alpha und IL-15, oder IFN-alpha und IL-21.

Vorzugsweise umfasst die Zusammensetzung einen pharmazeutisch akzeptablen Träger, vorzugsweise geeignet zur intrazellulären Verabreichung in einem Patienten, insbesondere Vehikel wie Liposomen oder Mikrosomen Formulierungen, welche insbesondere zur Verabreichung von Nukleinsäuren bevorzugt werden. Pharmazeutische Zusammensetzungen können pharmazeutisch geeignete Salze, zusätzlich Puffer, Tonizitätskomponenten oder pharmazeutisch geeignete Träger umfassen. Insbesondere inhibitorische Nukleinsäuren, wie antisense Nukleinsäuren, siRNA, shRNA, können in geeigneten therapeutischen Vektorsystemen vorgesehen werden. Pharmazeutische TrägerSubstanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzungen vorteilhaft.

Vorzugsweise umfasst das erfindungsgemäß zu verwendende Medikament Puffersubstanzen oder tonische Substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl, oder auch nichtionische Substanzen, wie zum Beispiel Glycerin oder Kohlenhydrate, umfassen.

Bevorzugterweise ist die erfindungsgemäß zu verwendende Zusammensetzung zur systemischen, topischen, oralen oder intranasalen Verabreichung geeignet hergerichtet. Diese Verabreichungsformen des Medikaments der vorliegenden Erfindung ermöglichen eine schnelle und unkomplizierte Aufnahme. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen. Weiters sind topische Verabreichungen vorgesehen. Insbesondere eine Verabreichung direkt in oder in die Nähe der Stelle, an der eine Immunantwort ausgelöst oder verstärkt werden soll, z.B. den Ort einer Infektion oder eines Tumors, hat den Vorteil, dass die NK-Zell-Aktivierung vordergründig am Zielort erfolgt.

Neben einer in vivo Verabreichung sind auch ex vivo Behandlungen von NK-Zellen möglich. Hierzu werden NK-Zellen aus einem Patienten isoliert ex vivo erfindungsgemäß behandelt und aktiviert und anschließend wieder in den Patienten zurückgeführt. Ein derartiges ex vivo Verfahren zur Behandlung von Zellen des Immunsystems wird beispielsweise in der WO 2009/073905 beschrieben, und kann umgelegt auf NK-Zellen erfindungsgemäß verwendet werden. Vorzugsweise erfolgt die ex vivo Variante des erfindungsgemäßen Verfahrens in Kombination mit den oben genannten zusätzlichen NK-Zell-Aktivatoren wie Immunzell-stimulierenden Zytokinen, insbesondere IL-2 und/oder IL-12, einem Interferon oder Interferon-Stimulans, einem Antikörper, oder einem TLR- oder PAMP-Rezeptor Liganden, vorzugsweise von TLR-2, TLR-7 oder TLR-8, sowie Kombinationen hiervon. Es ist möglich, dass entweder die Cbl-b Inhibition oder die zusätzlichen NK-Zell-Aktivatoren ex vivo an die NK-Zellen verabreicht werden und die weitere Aktivierung in vivo erfolgt, z.B. ex vivo Cbl-b Inbibition und in vivo Verabreichung des zusätzlichen NK-Zell-Aktivators, oder umgekehrt. Vorzugsweise können beide Schritte, Cbl-b Inhibition und zusätzliche NK-Zell-Aktivierung, ex vivo vorgenommen werden. Isolierte und aktivierte NK-Zellen können gezielt an den Zielort verabreicht werden. Die NK-Zellen können ex vivo isoliert werden. Zur spezifischen NK-Zell Aktivierung können ex vivo NK-Zellen in einem Zellisolat, z.B. PBMCs, angereichter oder daraus isoliert werden.

Die ex vivo Behandlung der NK Zellen kann auch ein Expandieren umfassen, vorzugsweise wie in US 7,435,596 B2 oder WO 2006/52534 A2 beschrieben. Hierzu können beispielsweise NK Zellen mit NK-Zell aktivierenden Zellen, welche den MHC-I komplex nur reduziert exprimieren und membrangebundenes IL-15 exprimieren, kontaktiert werden. Alternativ oder zusätzlich können NK Zellen mit IL-15 oder einem IL-15 Rezeptor Antikörper und einem CD137 Liganden oder einem CD137 Antikörper kontaktiert werden. Diese Expandierung wird optional in Kombination mit den genannten zusätzlichen NK-Zell-Aktivatoren wie Immunzell-stimulierenden Zytokinen vorgenommen.

Die vorliegende Erfindung wird weiters durch die folgenden Figuren und Beispiele dargestellt, ohne zwingend darauf beschränkt zu sein.

### Figuren:

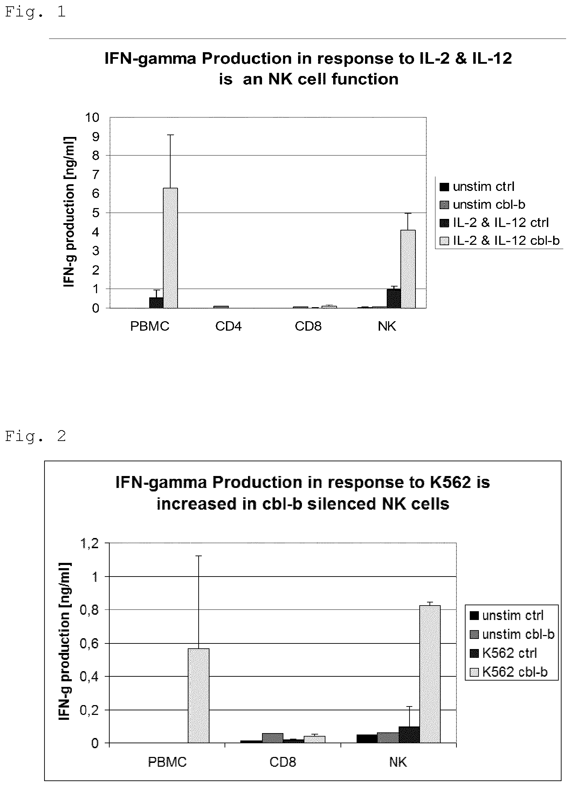

Fig 1: PBMCs bzw. daraus isolierte Zellfraktionen wurden mittels Cbl-b siRNA inhibiert und dann mit IL-2 und IL-12 stimuliert und die IFN-gamma Produktion nach 24h gemessen.
Fig. 2: PBMCs bzw. daraus isolierte Zellfraktionen wurden mittels Cbl-b siRNA inhibiert, mittels Koinkubation mit der K562 Tumorzelllinie stimuliert und die IFN-gamma Produktion nach 24h gemessen.

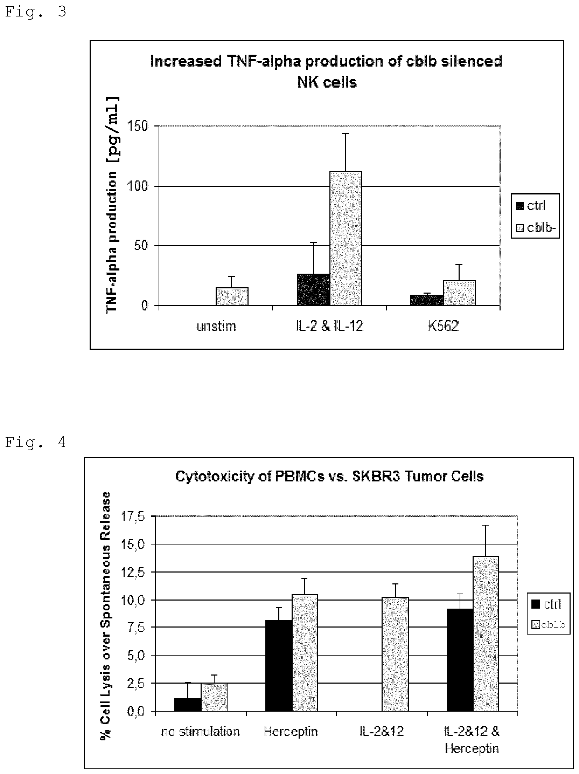

Fig. 3: Isolierte NK-Zellen wurden mittels Cbl-b siRNA inhibiert, und mittels K562 oder IL-2 und IL-12 stimuliert und die TNF-alpha Produktion nach 24h gemessen.
Fig. 4: PBMCs wurden mit Cbl-b siRNA inhibiert, und für 4h mit der Tumorzelllinie SKBR3 und wie angegeben zusätzlich unter Zugabe von Herceptin oder Zytokin inkubiert. Die Zytotoxizität wurde dann aufgrund des freigesetzten zellulären LDH-Enzyms bestimmt, welche mittels enzymatischen Assays gemessen wurde.

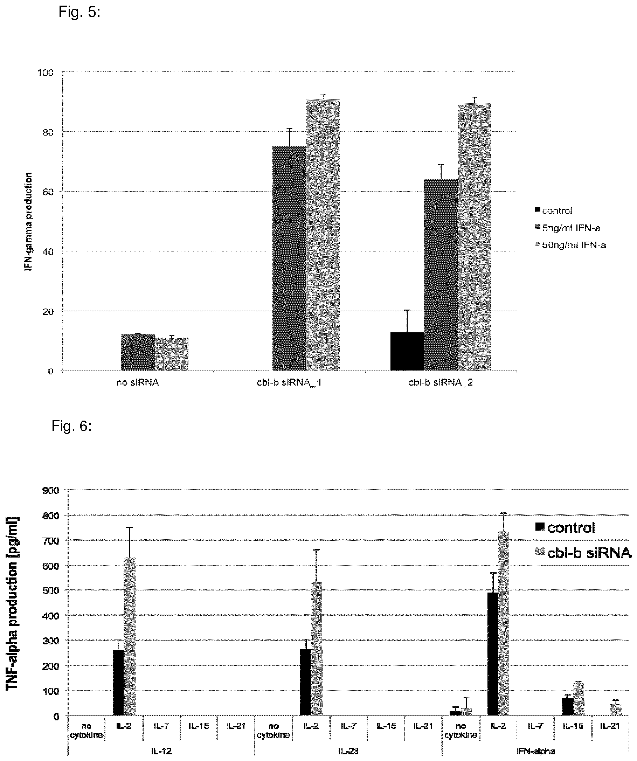

Fig. 5: Cbl-b Silencing in NK-Zellen erhöht die Reaktivtät gegenüber IFN-alpha Stimulation: NK-Zellen wurden mittels Cbl-b siRNA inhibiert, und mittels IFN- alpha stimuliert und die IFN-gamma Produktion nach 24h gemessen.
Fig. 6: Cbl-b Silencing in NK-Zellen erhöht die Reaktivtät gegenüber Kombinationen von Interleukinen und IFN-alpha: NK-Zellen wurden mittels Cbl-b siRNA inhibiert, und mittels IL-2, IL-7, IL-15 bzw. IL-21 entweder alleine oder zusammen mit IL-12, IL-23 oder IFN-alpha stimuliert und die TNF-alpha Produktion nach 24h gemessen.

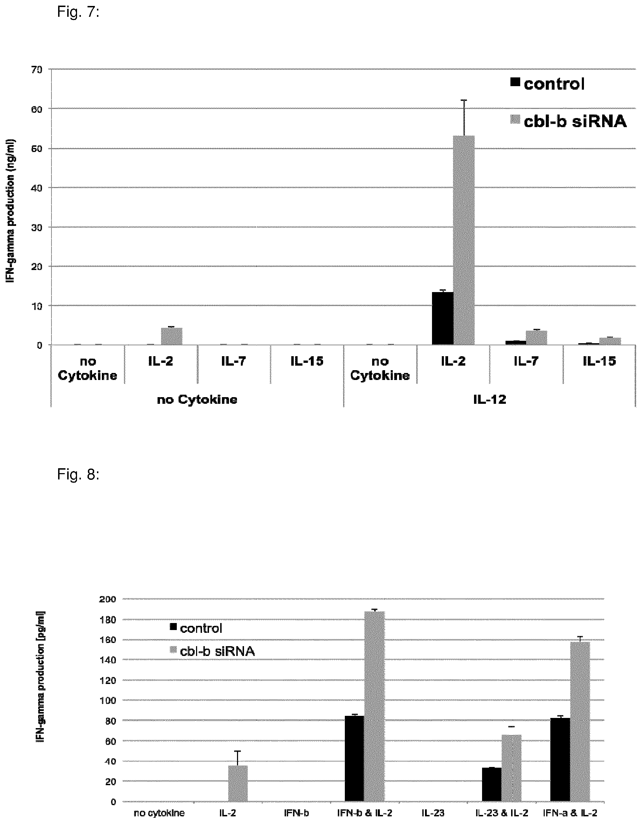

Fig. 7: Cbl-b Silencing in NK-Zellen erhöht die Reaktivität gegenüber Kombinationen von IL-12 mit verschiedenen common cytokine receptor gamma-chain Zytokinen: NK-Zellen wurden mittels Cbl-b siRNA inhibiert, und mittels IL-2, IL-7 bzw. IL-15 und entweder alleine oder zusammen mit IL-12 stimuliert und die IFN-gamma Produktion nach 24h gemessen.
Fig. 8: Cbl-b Silencing in NK-Zellen erhöht die Reaktivität nicht nur gegenüber Kombinationen von IL-2 mit IL-12, IL-23 und IFN-alpha oder IFN-beta: NK-Zellen wurden mittels Cbl-b siRNA inhibiert, und mittels IL-23, IFN- alpha oder IFN-beta entweder alleine oder in Gegenwart von IL-2 stimuliert und die IFN-gamma Produktion nach 24h gemessen.

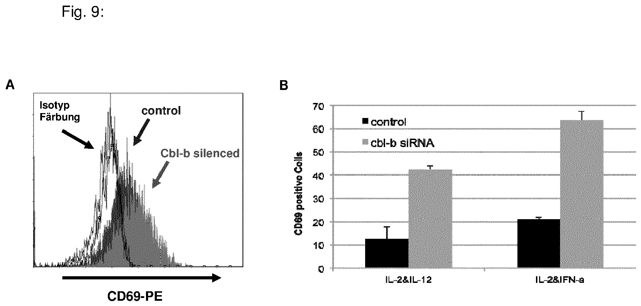

Fig. 9: Die erhöhte Reaktivität von NK-Zellen nach Cbl-b Silencing gegenüber Zytokin-Stimulation führt zu einer Erhöhung des Aktivierungsmarkers CD69 auf der Zelloberfläche: NK-Zellen wurden mittels Cbl-b siRNA inhibiert, und mittels IL-2 und entweder IFN- alpha oder IL-12 stimuliert und die CD69-Expression nach 48h bestimmt.

### Beispiel 1: Sequenzen

Die folgenden siRNA-Sequenzen wurden zur Inhibierung von Cbl-b verwendet:
A. Sense Sequenz:
   GUACUGGUCCGUUAGCAAAUU (SEQ ID Nr. 1)
      Antisense Sequenz:
   5'-PUUUGCUAACGGACCAGUACUU (SEQ ID Nr. 2)
B. Sense Sequenz:
   GGUCGAAUUUUGGGUAUUAUU (SEQ ID Nr. 3)
      Antisense Sequenz:
   5'-PUAAUACCCAAAAUUCGACCUU (SEQ ID Nr. 4)

### Beispiel 2: Cbl-b Inhibition in NK-Zellen

Mittels CPT Röhrchen (Vacutainer) wurde einem Spender Vollblut abgenommen und daraus PBMCs durch Zentrifugation abgetrennt. Aus PBMCs wurden NK-Zellen (inklusive der CD8⁺ CD3⁻ - Fraktion) und dann die CD8 und CD4 T-Zellen mittels magnetic selection isoliert. Mittels FACS wurde verifiziert, dass die Reinheit der entsprechenden Zellpopulationen zumindest 90% betrug. Sowohl die PBMCs als auch die daraus isolierten Zellfraktionen wurden mit siRNA Cbl-b unter Verwendung eines Amaxa Transfektionsgeräts transfiziert und mit rekombinantem humanem IL-2 (50ng/ml) und IL-12 (10ng/ml) über Nacht in Xvivo15 Medium stimuliert (Fig. 1). Die IFN-gamma Sekretion der so behandelten Zellen wurde dann mittels ELISA gemessen. Das Ergebnis zeigt klar, dass die stark erhöhte IFN-gamma Produktion von Cbl-b-inhibierten PBMCs nach IL-2 und IL-12 Stimulation eindeutig der Reaktion der NK-Zellen zugeordnet werden kann.

### Beispiel 3: Cbl-b Inhibition in NK-Zellen und Kostimulation

Eine Möglichkeit NK-Zellen zu kostimulieren ist über Tumorzelllinien, deren aberrante Oberflächenmarkerexpression nicht mehr das entsprechende Gleichgewicht zwischen inhibitorischen und aktivatorischen NK-Rezeptoren aufrechterhalten kann und die damit eine Aktivierung der NK-Zellen verursachen, beispielsweise die Tumorzelllinie K562. PBMCs sowie die CD8 und NK-Zellen wurden wie oben beschrieben isoliert und mit Cbl-b siRNA transfiziert. 1x10^5 dieser transfizierten Zellen wurden dann entweder alleine (unstim) oder mit jeweils 6x10^4 K562 Tumorzellen in Xvivo Medium über Nacht inkubiert und die IFN-gamma Sekretion wie oben bestimmt. Inkubation von Cbl-b inhibierten PBMCs mit dieser Tumorzellinie führte ebenfalls wieder zu einer starken IFN-gamma Produktion und wiederum konnte diese IFN-gamma Produktion klar auf den Beitrag der NK-Zellen zurückgeführt werden (Fig. 2). Beide Stimulationsmethoden führten auch zu einem Anstieg der TNF-alpha Produktion von NK-Zellen nach Cbl-b Inhibierung (Fig. 3).

### Beispiel 4: Tumorzytotoxizität durch Cbl-b Inhibition in NK-Zellen

Eine der Hauptfunktionen von NK-Zellen im Kontext der Tumorentstehung ist die direkte Zerstörung von Tumorzellen. Es wurde daher getestet, ob Cbl-b inhibierte PBMCs besser in der Lage sind Tumorzellen zu zerstören. Als Ziel- bzw. Target-Zelllinie wurde dabei die Her2-positive SKBR3 Brustkarzinomlinie verwendet, da in diesem Kontext auch der in der Tumortherapie eingesetzte Antikörper gegen Her2 getestet werden kann (Trastuzumab oder Herceptin). PBMCs wurden wieder wie oben beschrieben isoliert und mit Cbl-b siRNA transfiziert und entweder alleine oder mit 4x10^4 SKBR3-Zellen in Xvivo Medium für 4h inkubiert. Zusätzlich wurden in den mit Herceptin bezeichneten Konditionen 10µg/ml Herceptin-Antikörper zugegeben sowie in den mit IL-2 und IL-12 bezeichneten Konditionen wie oben beschrieben stimuliert. Die Zytotoxizität der PBMCs auf die SKBR3-Tumorzellen wurden dann durch die colorimetrische Messung der Freisetzung des Enzyms LDH aus dem Tumorzellzytosol bestimmt. Der spontane Release dieses Enzyms aus den PBMCs bzw. den Tumorzellen wurde, wie vom Colorimetrie-Messungskit Hersteller (Biovision) empfohlen, aus entsprechenden Einzelkontrollkonditionen bestimmt und abgezogen. Dabei zeigte sich, dass die Zelllyse durch Cbl-b-inhibierte Immunzellen grundsätzlich höher war als durch die mit Kontroll siRNA behandelten Zellen, wobei insbesondere die gleichzeitige Stimulation mit IL-2 und IL-12 zu einer signifikanten Erhöhung der Tumorzelllyse führte (Fig. 4).

Diese in vitro Ergebnisse zeigen daher, dass gleichzeitiges Inhibieren von Cbl-b in Zellen des adaptiven und des angeborenen Immunsystems ex vivo in unseparierten humanen PBMCs möglich ist, und führen weiters zur Schlussfolgerung, dass Cbl-b Inhibierung in NK-Zellen eine rationale Grundlage zur Kombination von Cbl-b Inhibierung mit Tumortherapien wie Gabe von rekombinantem IL-2 oder gegen Tumorantigene gerichteter therapeutischer Antikörper schafft.

### Beispiel 5: Cbl-b Silencing in NK-Zellen erhöht die Reaktivtät gegenüber IFN-alpha Stimulation

In PBMCs enthaltene NK-Zellen wurden wie in Beispiel 2 isoliert und mittels siRNA gesilenced. Die dabei verwendeten gegen Cbl-b gerichteten siRNA-Sequenzen waren:
Cbl-b siRNA 1:
   5' - CUCUAUUUGCGGAAUUA - 3' (SEQ ID Nr. 5)
   3' - AAUUCCGCAAAAUAGAGC- 5' (SEQ ID Nr. 6)
Cbl-b siRNA 2:
   5' - GUGAGAAUGAGUACUUUAAA - 3' (SEQ ID Nr. 7)
   3' - ACACUCUUACUCAUAAGAUU - 5' (SEQ ID Nr. 8)

Die Zellen wurden daraufhin entweder ohne Zytokin oder mit IFN-alpha über Nacht stimuliert (5 bzw. 50 ng/ml wie in Figur 5 angegeben) und die IFN-gamma Sekretion der so behandelten Zellen wurde dann mittels ELISA gemessen. Das Ergebnis zeigt klar, dass die Inhibition von Cbl-b in NK-Zellen zu einer stark erhöhten IFN-gamma Produktion führt (Figur 5). Die IFN-gamma Produktion von T- und NK-Zellen in der Leber ist als einer der kausalen Faktoren für die Wirksamkeit der IFN-alpha Therapie in der Behandlung chronischer Hepatitis-Infektionen definiert worden. Damit ist eine Rationale für eine Kombinationstherapie von Cbl-b Silencing mit systemischer IFN-alpha Therapie gegeben, insbesondere in den Fällen wo die Standardtherapie mit IFN-alpha alleine nicht zur vollständigen Heilung ausreicht.

Die Leber ist prinzipiell ein ideales Zielorgan für siRNA Therapien, da die Genexpression in der Leber durch systemische Gabe von siRNA gut veränderbar ist. Im gegenständlichen Fall ist daher sowohl eine Cbl-b Inhibition in NK-Zellen durch direkte systemische Gabe von Cbl-b spezifischer siRNA möglich als auch eine Zelltherapie durch Transfer von ex vivo gesilenceden NK-Zellen, da davon auszugehen ist, dass diese nach intravenöser Refundierung in ausreichendem Maße in den Bereich der erkrankten Leber migrieren.

### Beispiel 6: Cbl-b Silencing in NK-Zellen erhöht die Reaktivität gegenüber Kombinationen von IL-2 mit IFN-alpha oder IL-12/IL-23.

Da in den Beispielen 2 und 5 gezeigt werden konnte, dass Cbl-b Inhibition in NK-Zellen zu einer erhöhten Stimulierbarkeit durch IFN-alpha bzw. IL-2 und IL-12 führte, wurde eine systematischere Analyse der Stimulierbarkeit von Cbl-b gesilenceden NK-Zellen durchgeführt. IL-2 ist ein Mitglied der sogenannten "common cytokine receptor gamma-chain" Familie. Weitere Mitglieder dieser Familie sind u. a. IL-7, IL-15 und IL-21. IL-23 wiederum ist IL-12 in verschiedenen Hinsichten strukturell und funktionell ähnlich, und wird so wie IFN-alpha und IL-12 von aktivierten dendritischen Zellen produziert, die u. a. dadurch auch eine essentielle Rolle in der Aktivierung von NK-Zellen haben. Es wurden daher in diesem Experiment die NK-Zellen mit den common cytokine receptor gamma-chain Zytokinen IL-2, IL-7, IL-15 und IL-21 entweder alleine oder zusammen mit IL-12, IL-23 oder IFN-alpha stimuliert. Zum Vergleich wurden auch die DC-Zytokine IL-12, IL-23 und IFN-alpha ohne common cytokine receptor gamma-chain Zytokinen zugegeben. Dazu wurden die in PBMCs enthaltene NK-Zellen wie im Beispiel 5 isoliert und mittels siRNA gesilenced (mit Cbl-b siRNA 2) und die kontrollbehandelten bzw. Cbl-b gesilenceden NK-Zellen direkt miteinander verglichen. Die NK-Zellen wurden mit den Zytokinen wie angegeben über Nacht stimuliert (alle common cytokine receptor gamma-chain Zytokinen 50 ng/ml, die DC-Zytokine IL-12, IL-23 und IFN-alpha 10 ng/ml). Als Read-out wurde TNF- alpha gewählt (Bestimmung durch ELISA), da TNF- alpha ein Schlüsselzytokin in Immunreaktionen ist und seine Produktion nicht so IL-12 abhängig ist wie IFN- gamma. Die Ergebnisse in Fig. 6 zeigen, dass Cbl-b Inhibition von NK-Zellen zu einer wesentlich stärkeren Aktivierung und damit höheren Produktion von TNF-alpha führt. Besonders stark war die TNF-alpha Produktion, wenn die Cbl-b gesilenceden NK-Zellen mit Kombinationen von IL-2 und einem DC-Zytokin wie IL-12, IL-23 und IFN-alpha costimuliert wurden. Allerdings zeigte sich, dass auch andere common cytokine receptor gamma-chain Zytokine, insbesonders IL-15 in Gegenwart von IFN-alpha, zu einer wesentlich stärkeren Reaktion von Cbl-b gesilenceden NK-Zellen führten. Da die Produktion von DC-Zytokinen wie IL-12, IL-23 und IFN-alpha in vivo vor allem durch Bestandteile von Pathogenen die als TLR-Liganden wirken stimuliert wird, ist daher ein therapeutisches Rationale gegeben, die Cbl-b Inhibition von NK-Zellen entweder in der Situation chronischer Infektionen einzusetzen oder in der Therapie von Tumorerkankungen mit artifiziellen TLR-Liganden (bspw. TLR7/8-Liganden wie Imiquimod oder TLR9-Liganden wie CpG) einzusetzen. Den zusätzlichen Stimulus durch common cytokine receptor gamma-chain Zytokine IL-2 bzw. IL-15 in vivo kann man dabei bspw. durch Immunisierung mit Anti-Tumorvakzinen, die zu einer Produktion von IL-2 und IL-15 durch aktivierte antigenspezifische T-Zellen führen, oder durch direkte therapeutische Verabreichung an den Patienten vermitteln.

### Beispiel 7: Cbl-b Silencing in NK-Zellen erhöht die Reaktivität gegenüber Kombinationen von IL-12 mit verschiedenen common cytokine receptor gamma-chain Zytokinen.

Da im Beispiel 6 gezeigt werden konnte, dass Cbl-b Inhibition in NK-Zellen zu einer erhöhten Stimulierbarkeit durch verschiedene common cytokine receptor gamma-chain Zytokine führen kann, besonders wenn sie in Kombination mit üblicherweise DCproduzierten Zytokinen verwendet werden, wurde getestet, ob neben IL-2 auch IL-7 bzw. IL-15 zusammen mit IL-12, dem Schlüsselfaktor für die Induktion von IFN-gamma, zu einer erhöhten Produktion dieses Zytokins führen können. Dazu wurden die in PBMCs enthaltene NK-Zellen wie in Beispiel 5 und 6 isoliert, gesilenced und mit den Zytokinen IL-2 (50 ng/ml), IL-7 und IL-15 (je 20ng/ml) entweder alleine oder in Gegenwart von IL-12 (10ng/ml) wie angegeben über Nacht stimuliert. Die IFN-gamma Produktion wurde mittels ELISA bestimmt. Die Ergebnisse in Fig. 7 zeigen, dass die Cbl-b gesilenceden NK-Zellen zu einer wesentlich stärkeren Reaktion in Form von IFN-gamma Produktion auf alle 3 getesteten common cytokine receptor gamma-chain Zytokine in Gegenwart von IL-12 fähig sind. Ebenso zeigte sich, dass nur die Cbl-b gesilenceden NK-Zellen zu einer messbaren Produktion von IFN-gamma alleine aufgrund von IL-2 Stimulation in der Lage waren. IL-2 ist für die Therapie bestimmter maligner Tumorerkrankungen zugelassen, zeigt aber nur in einem geringen Teil der Patienten ausreichend Wirkung. Die Inhibition von Cbl-b in NK-Zellen ist demgemäß eine potentielle Strategie die Wirksamkeit von IL-2 in der Tumortherapie zu verbessern. Weiters sind Cbl-b gesilencede NK Zellen in der Lage wesentlich stärker auf die gleichzeitige Anwesenheit von IL12 und IL-7 bzw. IL-15 zu reagieren.

### Beispiel 8: Cbl-b Silencing in NK-Zellen erhöht die Reaktivität nicht nur gegenüber Kombinationen von IL-2 mit IL-12, IL-23 und IFN-alpha sondern auch mit IFN-beta.

Da im Beispiel 6 gezeigt wurde, dass Cbl-b Inhibition in NK-Zellen zu einer erhöhten Stimulierbarkeit durch IL-2 und IFN-alpha führt, wurde auch getestet, ob IFN-beta zu einer gleichartigen Wirkung fähig ist. Dazu wurden die in PBMCs enthaltene NK-Zellen wie in Beispiel 6 isoliert, gesilenced und mit den Zytokinen IL-23, IFN- alpha oder IFN-beta und entweder alleine oder in Gegenwart von IL-2 (50 ng/ml) wie angegeben über Nacht stimuliert. Die IFN-gamma Produktion wurde mittels ELISA bestimmt. Die Ergebnisse in Fig. 8 zeigen, dass die Cbl-b gesilenceden NK-Zellen zu einer wesentlich stärkeren Reaktion in Form von IFN-gamma Produktion auf alle 3 getesteten Zytokine in Gegenwart von IL-2 in der Lage sind.

### Beispiel 9: Die erhöhte Reaktivität von NK-Zellen nach Cbl-b Silencing gegenüber Zytokin-Stimulation führt zu einer Erhöhung des Aktivierungsmarkers CD69 auf der Zelloberfläche

Da in den Beispielen zuvor gezeigt wurde, dass Cbl-b gesilencede NK-Zellen durch erhöhte Zytokin-Produktion reagieren, wurde als weiterer Parameter die Expression des Aktivierungsmarkers CD69 auf der Zelloberfläche mittels FACS untersucht. Dazu wurden die in PBMCs enthaltenen NK-Zellen wie in Beispiel 6 isoliert, gesilenced und mit den Zytokinen IL-2 und entweder IFN-alpha oder IL-12 stimuliert. Nach 2 Tagen wurden die Zellen geerntet und die Oberflächenexpression von CD69 mittels FACS auf einem FC500-Zytometer untersucht. Zum Nachweis der Spezifizität des Stainings wurden die Zellen mittels CD56-PE-Cy5 und CD3-FITC gegengefärbt und mit CD69-PE oder einer entsprechenden Isotypkontrolle gefärbt (die Antikörper wurden gemäß der empfohlenen Arbeitsvorschrift des Herstellers Beckman-Coulter standardgemäß durchgeführt). Fig. 9A zeigt als Beispiel die erhöhte CD69-Expression von Cbl-b gesilenceden NK-Zellen im Vergleich zu kontrollbehandelten, die beide mit IFN- alpha und IL-2 behandelt wurden im Overlay-Histogramm. Fig. 9B fasst die quantitative Auswertung der CD69 Expression für Stimulation mit IL-2 und entweder IFN-alpha oder IL-12 zusammen. Dabei zeigt sich übereinstimmend mit den Daten der vorigen Beispiele, dass Cbl-b gesilencede NK-Zellen hyperreaktiv gegenüber der Stimulation mit diesen Zytokinen sind, und diese erhöhte Reaktivität sich in der verstärkten Aufregulation des Aktivierungsmarkers CD69 direkt zellulär manifestiert.

## Patentansprüche

1. Cbl-b Inhibitor ausgewählt aus inhibierenden Nukleinsäuren, die die Expression von Cbl-b reduzieren oder inhibieren, in Kombination mit mindestens einem Zytokin oder Interferon ausgewählt aus
• IL-2 und einem oder mehreren weiteren Zytokinen oder Interferonen ausgewählt aus IL-12, IL-23, IFN-alpha und IFN-beta, oder
• IFN-alpha und einem oder mehreren weiteren Zytokinen ausgewählt aus IL-15 und IL-21, oder
• IL-12 und IL-7, oder
• IL-12 und IL-15, ist,
zur Verwendung in der therapeutischen Behandlung von Krebs in einem Patienten umfassend die in vivo Verabreichung des Cbl-b Inhibitors an einen Patienten und/oder die ex vivo Verabreichung des Cbl-b Inhibitors an NK-Zellen des Patienten, wodurch die NK-Zellen immunaktiviert werden, und umfassend die in vivo Verabreichung der Zytokine und/oder Interferone an einen Patienten und/oder die ex vivo Verabreichung der Zytokine und/oder Interferone an NK-Zellen des Patienten, und umfassend die Rückführung von ex vivo behandelten NK-Zellen in den Patienten.

2. Cbl-b Inhibitor Kombination zur Verwendung nach Anspruch 1, **dadurch gekennzeichnet, dass** der Cbl-Inhibitor ausgewählt ist aus antisense Oligonukleotiden, siRNA oder shRNA.

3. Cbl-b Inhibitor Kombination zur Verwendung nach einem der Ansprüche 1 oder 2, **dadurch gekennzeichnet, dass** der Cbl-b Inhibitor in Kombination mit IL-2 und IL-12, IL-2 und IFN-beta, IL-2 und IL-23, oder IL-2 und IFN-alpha verabreicht wird.

4. Cbl-b Inhibitor Kombination zur Verwendung nach einem der Ansprüche 1 oder 2, **dadurch gekennzeichnet, dass** der Cbl-b Inhibitor in Kombination mit IL-12 und einem oder mehreren weiteren Zytokinen ausgewählt aus IL-2, IL-15 und IL-7 verabreicht wird.

5. Cbl-b Inhibitor Kombination zur Verwendung nach einem der Ansprüche 1 bis 4, **dadurch gekennzeichnet, dass** der Patient ausgewählt ist aus einem Säugetier oder einem Menschen.

6. Pharmazeutische Zusammensetzung umfassend einen Cbl-b Inhibitor ausgewählt aus inhibierenden Nukleinsäuren, die die Expression von Cbl-b reduzieren oder inhibieren, und
• IL-2 und ein oder mehrere weitere Zytokine oder Interferone ausgewählt aus IL-12, IL-23, IFN-alpha und IFN-beta, oder
• IFN-alpha und ein oder mehrere weitere Zytokine ausgewählt aus IL-15 und IL-21, oder
• IL-12 und IL-7, oder
• IL-12 und IL-15,
zur Verwendung in der therapeutischen Behandlung von Krebs in einem Patienten.

7. Zusammensetzung nach Anspruch 6, **dadurch gekennzeichnet, dass** der Cbl-Inhibitor ausgewählt aus antisense Oligonukleotiden, siRNA oder shRNA ist.

8. Zusammensetzung nach Anspruch 6 oder 7, umfassend IL-2 und IL-12, oder IL-2 und IFN-beta, oder IL-2 und IL-23, oder IL-12 und IL-7, oder IL-12 und IL-15, oder IFN-alpha und IL-2, oder IFN-alpha und IL-15, oder IFN-alpha und IL-21.

9. Zusammensetzung nach einem der Ansprüche 6 bis 8 umfassend einen pharmazeutisch akzeptablen Träger.

10. Zusammensetzung nach Anspruch 9, **dadurch gekennzeichnet dass** der pharmazeutisch akzeptable Träger zur intrazellulären Verabreichung in einem Patienten geeignet ist.

11. Zusammensetzung nach Anspruch 9 oder 10, **dadurch gekennzeichnet, dass** der pharmazeutisch akzeptable Träger eine Liposomen- oder Mikrosomen-Formulierung ist.

## Claims

1. Cbl-b inhibitor, selected from inhibitory nucleic acids which reduce or inhibit the expression of Cbl-b, in combination with at least one cytokine or interferon selected from
• IL-2 and one or more further cytokines or interferons selected from IL-12, IL-23, IFN-alpha and IFN-beta, or
• IFN-alpha and one or more further cytokines selected from IL-15 and IL-21, or
• IL-12 and IL-7, or
• IL-12 and IL-15,
for use in the therapeutic treatment of cancer in a patient, comprising the *in vivo* administration of said Cbl-b inhibitor to a patient and/or the ex *vivo* administration of said Cbl-b inhibitor to NK cells obtained from the patient, thereby immune-activating said NK cells, and comprising the *in vivo* administration of said cytokines and/or interferons to a patient and/or the ex *vivo* administration of said cytokines and/or interferons to NK cells obtained from the patient, and comprising re-introducing ex *vivo-*treated NK cells into the patient.

2. Cbl-b inhibitor combination for use according to claim 1, **characterized in that** the Cbl-b inhibitor is selected from antisense oligonucleotides, siRNA, or shRNA.

3. Cbl-b inhibitor combination for use according to any one of claims 1 or 2, **characterized in that** the Cbl-b inhibitor is administered in combination with IL-2 and IL-12, IL-2 and IFN-beta, IL-2 and IL-23, or IL-2 and IFN-alpha.

4. Cbl-b inhibitor combination for use according to any one of claims 1 or 2, **characterized in that** the Cbl-b inhibitor is administered in combination with IL-12 and one or more further cytokines selected from IL-2, IL-15, and IL-7.

5. Cbl-b inhibitor combination for use according to any one of claims 1 to 4, **characterized in that** the patient is selected from a mammal or a human being.

6. Pharmaceutical composition comprising a Cbl-b inhibitor selected from inhibitory nucleic acids which reduce or inhibit the expression of Cbl-b, and
• IL-2 and one or more further cytokines or interferons selected from IL-12, IL-23, IFN-alpha and IFN-beta, or
• IFN-alpha and one or more further cytokines selected from IL-15 and IL-21, or
• IL-12 and IL-7, or
• IL-12 and IL-15,
for use in the therapeutic treatment of cancer in a patient.

7. Composition according to claim 6, **characterized in that** the Cbl-b inhibitor is selected from antisense oligonucleotides, siRNA, or shRNA.

8. Composition according to claim 6 or 7, comprising IL-2 and IL-12, or IL-2 and IFN-beta, or IL-2 and IL-23, or IL-12 and IL-7, or IL-12 and IL-15, or IFN-alpha and IL-2, or IFN-alpha and IL-15, or IFN-alpha and IL-21.

9. Composition according to any one of claims 6 to 8, comprising a pharmaceutically acceptable carrier.

10. Composition according to claim 9, **characterized in that** the pharmaceutically acceptable carrier is suitable for intracellular administration in a patient.

11. Composition according to claim 9 or 10, **characterized in that** the pharmaceutically acceptable carrier is a liposomal or microsomal formulation.

## Revendications

1. Inhibiteur de Cbl-b, choisi parmi les acides nucléiques inhibants, qui réduisent ou inhibent l'expression de Cbl-b, en combinaison avec au moins une cytokine ou un interféron choisi parmi
- IL-2 et une ou plusieurs cytokines ou un ou plusieurs interférons supplémentaires choisis parmi IL-12, IL-23, IFN-alpha et IFN-bêta, ou
- IFN-alpha et une ou plusieurs cytokines supplémentaires choisies parmi IL-15 et IL-21, ou
- IL-12 et IL-7, ou
- IL-12 et IL-15,
pour une utilisation dans le traitement thérapeutique du cancer chez un patient, comprenant l'administration in vivo de l'inhibiteur de Cbl-b à un patient et/ou l'administration ex vivo de l'inhibiteur de Cbl-b à des cellules NK du patient, ce en conséquence de quoi il y a une immuno-activation des cellules NK, et comprenant l'administration in vivo des cytokines et/ou des interférons à un patient et/ou l'administration ex vivo des cytokines et/ou des interférons à des cellules NK du patient, et comprenant le renvoi, dans les patients, des cellules NK traitées ex vivo.

2. Combinaison d'un inhibiteur de Cbl-b pour l'utilisation selon la revendication 1, **caractérisée en ce que** l'inhibiteur de Cbl-b est choisi parmi les oligonucléotides antisens, le pARNi ou le shRNA (petit ARN en épingle à cheveux).

3. Combinaison d'un inhibiteur de Cbl-b pour l'utilisation selon l'une des revendications 1 ou 2, **caractérisée en ce que** l'inhibiteur de Cbl-b est administré en combinaison avec IL-2 et IL-12, IL-2 et IFN-bêta, IL-2 et IL-23, ou IL-2 et IFN-alpha.

4. Combinaison d'un inhibiteur de Cbl-b pour l'utilisation selon l'une des revendications 1 ou 2, **caractérisée en ce que** l'inhibiteur de Cbl-b est administré en combinaison avec IL-12 et une ou plusieurs cytokines supplémentaires choisies parmi IL-2, IL-15 et IL-7.

5. Combinaison d'un inhibiteur de Cbl-b pour l'utilisation selon l'une des revendications 1 à 4, **caractérisée en ce que** le patient est choisi parmi un mammifère ou un humain.

6. Composition pharmaceutique comprenant un inhibiteur de Cbl-b choisi parmi les acides nucléiques inhibants, qui réduisent ou inhibent l'expression de Cbl-b, et
- IL-2 et une ou plusieurs cytokines ou un ou plusieurs interférons supplémentaires choisis parmi IL-12, IL-23, IFN-alpha et IFN-bêta, ou
- IFN-alpha et une ou plusieurs cytokines supplémentaires choisies parmi IL-15 et IL-21, ou
- IL-12 et IL-7, ou
- IL-12 et IL-15,
pour une utilisation dans le traitement thérapeutique du cancer chez un patient.

7. Composition selon la revendication 6, **caractérisée en ce que** l'inhibiteur de Cbl-b est choisi parmi les oligonucléotides antisens, le pARNi ou le shRNA (petit ARN en épingle à cheveux).

8. Composition selon la revendication 6 ou 7, comprenant IL-2 et IL-12, ou IL-2 et IFN-bêta, ou IL-2 et IL-23, ou IL-12 et IL-7, ou IL-12 et IL-15, ou IFN-alpha et IL-2, ou IFN-alpha et IL-15, ou IFN-alpha et IL-21.

9. Composition selon l'une des revendications 6 à 8, comprenant un support pharmaceutiquement acceptable.

10. Composition selon la revendication 9, **caractérisée en ce que** le support pharmaceutiquement acceptable convient à une administration intracellulaire chez un patient.

11. Composition selon la revendication 9 ou 10, **caractérisée en ce que** le support pharmaceutiquement acceptable est une formulation de liposomes ou de microsomes.


### SEQUENCE LISTING

<110> APEIRON BIOLOGICS AG
<120> Verfahren zur Aktivierung von NK-Zellen
<130> r60068
<150> EP10197146.3
   <151> 2010-12-28
<160> 8
<170> BiSSAP 1.0
<210> 1
   <211> 21
   <212> RNA
   <213> Artificial Sequence
<220>
   <221> source
   <222> 1..21
   <223> /mo1_type="RNA" /note="siRNA" /organism="Artificial Sequence"
<400> 1
   guacuggucc guuagcaaau u 21
<210> 2
   <211> 21
   <212> RNA
   <213> Artificial Sequence
<220>
   <221> source
   <222> 1..21
   <223> /mol_type="RNA" /note="siRNA" /organism="Artificial Sequence"
<400> 2
   uuugcuaacg gaccaguacu u 21
<210> 3
   <211> 21
   <212> RNA
   <213> Artificial Sequence
<220>
   <221> source
   <222> 1..21
   <223> /mol_type="RNA" /note="siRNA" /organism="Artificial Sequence"
<400> 3
   ggucgaauuu uggguauuau u 21
<210> 4
   <211> 21
   <212> RNA
   <213> Artificial Sequence
<220>
   <221> source
   <222> 1..21
   <223> /mol_type="RNA" /note="siRNA" /organism="Artificial Sequence"
<400> 4
   uaauacccaa aauucgaccu u 21
<210> 5
   <211> 17
   <212> RNA
   <213> artificial sequences
<220>
   <221> source
   <222> 1..17
   <223> /mol_type="RNA" /note="siRNA" /organism="artificial sequences"
<400> 5
   cucuauuugc ggaauua 17
<210> 6
   <211> 18
   <212> RNA
   <213> artificial sequences
<220>
   <221> source
   <222> 1..18
   <223> /mol_type="RNA" /note="siRNA" /organism="artificial sequences"
<400> 6
   cgagauaaaa cgccuuaa 18
<210> 7
   <211> 20
   <212> RNA
   <213> artificial sequences
<220>
   <221> source
   <222> 1..20
   <223> /mol_type="RNA" /note="siRNA" /organism="artificial sequences"
<400> 7
   gugagaauga guacuuuaaa 20
<210> 8
   <211> 20
   <212> RNA
   <213> artificial sequences
<220>
   <221> source
   <222> 1..20
   <223> /mol_type="RNA" /note="siRNA" /organism="artificial sequences"
<400> 8
   uuagaauacu cauucucaca 20